# EUROPÄISCHE PATENTSCHRIFT

(11) **EP 3 876 890 B1**
(45) Veröffentlichungstag und Bekanntmachung des Hinweises auf die Patenterteilung: **01.01.2025**
(21) Anmeldenummer: 19795565.1
(22) Anmeldetag: 31.10.2019
(51) Int. Cl.: A61G 12/00, A61G 13/10

(54) **TRAGARMSYSTEM FÜR EIN MEDIZINISCHES GERÄT, VERFAHREN ZUM BETREIBEN EINES TRAGARMSYSTEM SOWIE VERFAHREN ZUM GESTALTEN EINES TRAGARMSYSTEMS**
SUPPORT ARM SYSTEM FOR A MEDICAL DEVICE, METHOD FOR OPERATING A SUPPORT ARM SYSTEM AND A METHOD FOR FORMING A SUPPORT ARM SYSTEM
SYSTÈME DE BRAS DE SUPPORT POUR UN APPAREIL MÉDICAL, PROCÉDÉ DE FONCTIONNEMENT D'UN SYSTÈME DE BRAS DE SUPPORT AINSI QUE PROCÉDÉ DE CONSTRUCTION D'UN SYSTÈME DE BRAS DE SUPPORT

(30) Priorität: 05.11.2018 EP 18204371
(43) Veröffentlichungstag der Anmeldung: 15.09.2021
(73) Patentinhaber: Ondal Medical Systems GmbH, 36088 Hünfeld (DE)
(72) Erfinder: TRAPP, Markus, 36282 Hauneck Rotensee (DE); PFORR, André, 36088 Hünfeld (DE)
(74) Vertreter: Graf von Stosch Patentanwaltsgesellschaft mbH
(86) Internationale Anmeldenummer: PCT/EP2019/079935
(87) Internationale Veröffentlichungsnummer: WO 2020/094518

(56) Entgegenhaltungen:
- EP-A1- 2 671 557
- EP-A1- 2 873 403
- EP-A2- 2 684 549
- EP-A2- 3 372 189
- WO-A1-2017/125677

## Beschreibung

Die vorliegende Erfindung betrifft ein Tragarmsystem zum bewegbaren Halten wenigstens eines medizinischen Geräts, ein Verfahren zum Betreiben eines Tragarmsystems sowie ein Verfahren zum Gestalten eines Tragarmsystems.

Tragarmsysteme werden beispielsweise in Operationssälen zum örtlichen verlagerbaren Halten einer medizintechnischen Einrichtung genutzt. Beispielsweise kann dazu ein Tragarm beweglich mittels einer Lageranordnung an einer Decke, einer Wand oder einem Boden verankert sein. Auch eine Befestigung auf einem Gestell mit Rollen ist möglich. Tragarmsysteme weisen üblicherweise wenigstens einen beweglichen Tragarm zum Halten des medizinischen Geräts, welches auch als medizintechnisches Gerät bezeichnet werden kann, beispielsweise einer OP-Lampe, OP-Besteck oder zahnmedizinische Bohrer, auf. Ebenso können jeweilige Tragarmsysteme auch wenigstens eine Bremsvorrichtung umfassen, welche dazu ausgebildet ist, eine Stellung des Tragarms und damit des medizinischen Geräts festzulegen und/oder jeweilige Bewegungen des Tragarms und damit des medizinischen Geräts zu hemmen. Das örtliche Verlagern medizinischer Geräte ist bei vielen medizinischen Prozeduren essentiell, um dem medizinischen Personal die Arbeit zu erleichtern oder überhaupt zu ermöglichen. Ebenso muss aber auch ein ungewolltes Verschieben des Geräts oder gar ein Bruch des Tragarmsystems unbedingt vermieden werden, um nicht den Patienten potenziell zu gefährden.

Beispielsweise ist aus der WO 2016/026547 ein als Stativvorrichtung bezeichnetes Tragarmsystems bekannt, bei welchem eine auf die Stativvorrichtung einwirkende Kraft erfasst und in Abhängigkeit davon eine Bremse für einen Tragarm aktiviert und deaktiviert wird. Damit kann eine Position einer medizintechnischen Einrichtung selbsttätig festgelegt bzw. deren Verstellung freigegeben werden. Durch diese zusätzlichen Komponenten ist das Tragarmsystem besonders komfortabel und sicher bei der Benutzung. Auch hier muss natürlich die Sicherheit für jeweilige Patienten und auch die Stativvorrichtung benutzendes medizinisches Personal aufwendig sichergestellt werden.

Dies bedingt große Sicherheitsmargen bei der Konstruktion solcher Tragarmsysteme hinsichtlich deren Belastung und häufige sowie umfangreiche Wartungsarbeiten. Dadurch sind entsprechende Tragarmsysteme sowohl in Anschaffung als auch Unterhalt sehr teuer. Damit verbunden ist entweder ein hoher Preis medizinischer Behandlungen; oder entsprechende Tragarmsysteme stehen überhaupt nicht zur Verfügung, weswegen die medizinischen Behandlungsmöglichkeiten eingeschränkt sein können, gerade in kleinen Krankenhäusern oder Ambulatorien. Alternativ können auch lediglich simple und damit kostengünstige Tragarmsysteme zur Verfügung gestellt werden, wodurch jedoch jeweilige Behandlungen für das medizinische Personal aufwendiger durchzuführen sind.

EP 3372189 A2 beschreibt ein Tragarmsystem zum bewegbaren Halten eines medizinischen Geräts mit einem bewegbar gelagerten Tragarm, welcher zum Halten des medizinischen Geräts ausgebildet ist. Dieses System umfasst eine Belastungserfassungsvorrichtung zum Erfassen jeweiliger Belastungsdaten des Tragarmsystems durch dessen Benutzung.

Aufgabe der vorliegenden Erfindung ist es, die Kosten eines Tragarmsystems für medizinisches Gerät zu senken.

Diese Aufgabe wird erfindungsgemäß durch die Gegenstände der unabhängigen Patentansprüche gelöst. Vorteilhafte Ausgestaltungen mit zweckmäßigen Weiterbildungen der Erfindung sind in den jeweiligen Unteransprüchen angegeben, wobei vorteilhafte Ausgestaltungen eines Aspekts als vorteilhafte Ausgestaltungen jeweiliger anderer Aspekte und umgekehrt anzusehen sind.

Ein erster Aspekt der Erfindung betrifft ein Tragarmsystem zum bewegbaren Halten wenigstens eines medizinischen Geräts. Das Tragarmsystem umfasst wenigstens einen bewegbar gelagerten Tragarm, welcher zum Halten des wenigstens einen medizinischen Geräts ausgebildet ist. Eine Verbindung zwischen dem Tragarm und dem medizinischen Gerät kann fest oder lösbar sein und das Gerät auch Teil des Systems sein. Vorzugsweise umfasst das Tragarmsystem eine Lageranordnung zum bewegbaren Lagern des Tragarms, beispielsweise mit einem Gleit- oder Rollenlager, mit einem Drehgelenk und/oder einer Führungsschiene. Mittels der Lageranordnung kann der Tragarm verankert werden, beispielsweise an einer Wand, Decke, einem Rollengestell und/oder auch weiteren Tragarmen. Entsprechend können auch mehrere Lager vorgesehen sein. Die Lageranordnung kann auch eine Bremsvorrichtung umfassen, um eine Bewegung des Tragarms einstellbar zu hemmen und/oder diesen wählbar in einer Position festzulegen. Der wenigstens eine Tragarm kann insbesondere rotatorisch und/oder translatorisch bewegbar gelagert sein, mit einem oder mehreren Freiheitsgraden der Bewegung. Eine bewegbare Lagerung des Tragarms kann auch zwei teleskopisch miteinander verbundene Tragarme betreffen. Weitere Tragarme können nur mittelbar zum Halten des medizinischen Geräts ausgebildet sein, indem sie beispielsweise den haltenden Tragarm mit einer Verankerung verbinden und so zusätzliche Freiheitsgrade zur Verfügung stellen.

Weiterhin weist das Tragarmsystem wenigstens einer Belastungserfassungsvorrichtung auf, welche zum Erfassen jeweiliger Belastungsdaten des Tragarmsystems durch dessen Benutzung, insbesondere durch ein Bewegen jeweiliger Tragarms, ausgebildet ist. Die Benutzung kann aber auch ein stationäres Halten, wobei hier auch Zeitdauer und/oder eine Last erfasst werden kann, betreffen. Üblicherweise kann unter Benutzung eine Benutzung des medizinischen Geräts, beispielsweise für eine medizinische Behandlung, verstanden werden, welche dann eine Belastung des Tragarms durch Bewegen und Nutzung des medizinischen Geräts bewirkt. Die erfasste Belastung bzw. die Belastungsdaten kann beispielsweise Rückschlüsse auf einen Verschleiß und/oder eine Beschädigung des Tragarmsystems erlauben, wodurch eine Wartung zielgerichtet erfolgen kann und/oder auch die Sicherheit des Tragarmsystems besonders hoch sein kann, da beispielsweise auch nicht sichtbare Beschädigung, wie Materialermüdung, rechtzeitig anhand der Belastungsdaten erkannt werden können. Die Belastungsdaten können beispielsweise einer Gesamtbelastung des Systems entsprechen, welche auch als aggregierte Belastung bezeichnet werden kann. Alternativ oder zusätzlich kann auch jeweils die getrennte Belastung einzelner Komponenten erfasst werden, um diese zielgerichtet warten und/oder austauschen zu können. Beispielsweise kann eine Tragarmbelastung jeweiliger Tragarm und/oder eine Lagerbelastung jeweiliger Lageranordnungen getrennt erfasst werden. Jeweilige Belastungen können bevorzugt nur extern sein, also auf einer äußeren Krafteinwirkung beruhen, und insbesondere strukturelle Belastungen einzelner Komponenten und Gelenke sein.

Weiterhin weist das Tragarmsystem wenigstens eine Speichervorrichtung auf, welche zum Speichern der erfassten Belastungsdaten ausgebildet ist. Dadurch können die Belastungsdaten zur späteren Analyse bereitstehen. Insbesondere kann der Hersteller des Tragarmsystems von einem oder mehreren Systemen Belastungsdaten sammeln und anhand dieser Daten eine überarbeitete Konstruktion auslegen, sodass diese besonders kostengünstig, leicht und wartungsarm sein kann. Die Belastungserfassungsvorrichtung und die Speichervorrichtung können dabei zur Datenübertragung miteinander gekoppelt sein, beispielsweise per Funk oder kabelgebunden.

Die Speicherung ist dabei im Gegensatz zu einer Erfassung von auf das Tragarmsystem einwirkenden Kräften zu sehen, welche dann lediglich zur Steuerung einer Komponente genutzt werden. Beispielsweise kann mittels Teilen der erfassten Belastungsdaten auch eine Bremsvorrichtung des Tragarmsystems gesteuert werden, beispielsweise um dessen Bewegung bedarfsgerecht zu hemmen oder freizugeben, wobei diese Daten dann jedoch ohne Speicherung nicht zur weiteren Auswertung und/oder anderweitigen Nutzung zur Verfügung stehen. Allerdings kann das Tragarmsystem Sensoren und/oder Erfassungseinrichtungen zum Steuern der Bremsvorrichtung auch zum Erfassen der Belastungsdaten nutzen, sodass auf zusätzliche teure Sensoren verzichtet werden kann.

Die Speichervorrichtung stellt also bevorzugt nicht nur einen temporären Speicher für Datenübermittlung und/oder Steuerung bzw. Verarbeitung in einer Steuereinrichtung dar. Stattdessen ist die Speichervorrichtung bevorzugt für ein langfristiges und dauerhaftes Aufbewahren jeweiliger Belastungsdaten ausgebildet. Beispielsweise kann die Speichervorrichtung einen RAM-Speicher, eine Festplatte und/oder ein optisches Laufwerk aufweisen. Die Speichervorrichtung kann auch eine Schnittstelle zur Datenübermittlung an systemexterne Komponenten umfassen. Die Belastungsdaten können insbesondere einem zeitlichen Verlauf der Belastung des Tragarmsystems bzw. dessen jeweiligen Komponenten entsprechen. Insbesondere kann zusammen mit den Belastungsdaten auch eine jeweilige Benutzungsart erfasst und gespeichert werden, beispielsweise die dabei durchgeführte medizinische Behandlung.

Mit dem Tragarmsystem und den gespeicherten Belastungsdaten ist es so möglich abzuleiten, wann eine Wartung und/oder ein Ersatz notwendig ist, insbesondere jeweiliger spezifischer Komponenten. Bei Erneuerungsprojekten eines solchen Tragarmsystems kann anhand der erfassten Belastungsdaten ein besonders bedarfsgerecht ausgelegtes und damit kostengünstiges Nachfolgetragarmsystem zur Verfügung gestellt werden. Auch im Rahmen jeweiliger Produktentwicklungen können jeweilige gespeicherte Lastkollektive bei verschiedenen Anwendungen berücksichtigt werden. Bei jeweiligen Reklamationen kann zudem durch den Hersteller anhand jeweiliger Belastungsdaten gegebenenfalls nachgewiesen werden, dass das Tragarmsystem fehlbedient und/oder missbräuchlich belastet wurde, um gegebenenfalls Regressforderungen abwehren zu können. Auch dadurch kann das System besonders kostengünstig sein, da das Risiko solcher Forderungen nicht mehr im Kaufpreis berücksichtigt werden müssen. Zudem können jeweilige Belastungsdaten dazu genutzt werden, Fehlbedienungen zu erkennen und jeweiliges Personal in der entsprechenden korrekten Benutzung zu schulen, um Verschleiß zu reduzieren. Auch eine Auslegung, die einen entsprechenden Missbrauch und/oder Fehlbedienungen unmöglich macht, beispielsweise durch entsprechende Auswahl anderer und/oder zusätzlicher Freiheitsgrade ist so möglich.

Das Tragarmsystem ermöglicht es, den Nachteil herkömmlicher System zu überwinden. Nachteilig war insbesondere, dass keine Aussage hinsichtlich der tatsächlichen Beanspruchung eines Tragarmsystems möglich war. So kann vermieden werden, dass ein Tragarmsystem in einem Raum befestigt ist und nicht oder nur mit intensiver, aufwendiger Inspektion festgestellt werden kann, ob das Tragarmsystem stark beansprucht und Verschleißerscheinungen aufweist, ob es missbräuchlich genutzt wurde und beschädigt ist oder ob es kaum bewegt wurde und daher nahezu die Funktionalität eines Neugeräts hat. Auch hierdurch können allfällige Kosten gesenkt werden, da anhand der Belastungsdaten der Zustand des Tragarmsystems nachgewiesen werden kann und so bei einem Verkauf eines gebrauchten Systems ein höherer Preis erzielt werden könnte. Auch für Nutzer, wie ein Krankenhaus, ist in der Benutzung eine Effizienzsteigerung möglich, da Ausfallrisiken gesenkt werden können und/oder eine höhere Verfügbarkeit des Systems sichergestellt werden kann. Dadurch kann gegebenenfalls auch auf sonst notwendige Reservesysteme, welche zudem einen Lagerraum benötigen, ebenso wie auf umfangreiche Beschaffung und Lagerung von Ersatzteilen, verzichtet werden.

In weiterer vorteilhafter Ausgestaltung des Tragarmsystems ist es vorgesehen, dass das Tragarmsystem ferner eine Auswertvorrichtung umfasst, welche dazu ausgebildet ist, in Abhängigkeit von den gespeicherten Belastungsdaten allfällige Wartungserfordernisse für das Tragarmsystem zu bestimmen. Damit können automatisch allfällige Wartungen veranlasst werden. Die Wartungserfordernisse können insbesondere in Abhängigkeit von einem zeitlichen Verlauf der Belastung und/oder einer aggregierten Belastung bestimmt werden. Wartungserfordernisse können beispielsweise den Austausch oder die Reparatur von dem Tragarmsystem oder einzelnen Komponenten, wie beispielsweise Bremsbeläge, Lager oder andere Verschleißteile, betreffen, ein Nachziehen von Verbindungen wie Verschraubungen, ein Schmieren von Lagerungen, und/oder die Fälligkeit einer Inspektion.

Alternativ oder zusätzlich kann die Auswertvorrichtung dazu ausgebildet sein, in Abhängigkeit von den gespeicherten Belastungsdaten strukturelle Anforderungen an die Auslegung eines weiteren Tragarmsystems vorzugsweise für den gleichen Einsatz zu bestimmen, insbesondere in Abhängigkeit von einem zeitlichen Verlauf der Belastung und/oder einer aggregierten Belastung. Die Auswertvorrichtung kann dazu ausgebildet sein, die strukturellen Anforderungen an die Auslegung, beispielsweise durch die Wahl einer entsprechenden Tragarmlänge, Materialauswahl der Strukturkomponenten, der Wandstärke und/oder des Lager(typs) zu definieren. Damit können jeweilige Konstruktionen von Tragarmsystemen optimiert werden und/oder neue Entwürfe aufgrund empirisch umfangreich ermittelter Belastungsdaten gestaltet werden. Dabei kann auch zunächst ein Testtragarmsystem zum Einsatz kommen, um dann das tatsächliche System in Abhängigkeit der von dem Testtragarmsystem gesammelten Testbelastungsdaten zu ermitteln. Das später installierte Tragarmsystem muss dabei nicht mehr zwingend die Komponenten zum Erfassen und Speichern der Belastungsdaten aufweisen. Insbesondere bei der Bestimmung von strukturellen Anforderungen ist es vorteilhaft, wenn auch die Art der Belastung erfasst und gespeichert werden kann. Dann können bei anderen Benutzungsprofilen, wie beispielsweise einem Krankenhaus mit anderem Operationsschwerpunkt, dennoch die strukturellen Anforderungen anhand geeignet extrapolierter Belastungen auf Basis der den gespeicherten, gerätespezifischen Belastungsarten zugeordneten Belastungsdaten sehr genau bestimmt werden.

Bei den strukturellen Anforderungen kann es sich um Belastungen handeln, welchen ein andersartiges oder gleiches Tragarmsystem widerstehen können soll. Beispielsweise kann die Auswertvorrichtung eine notwendige Widerstandsfähigkeit eines Tragarmsystems mit anderen Komponenten, wie Gelenken oder anderen Teilen, anderen Freiheitsgraden und/oder unterschiedlich langen Tragarmen aufgrund der Belastungsdaten berechnen, um dieses auszulegen. Diese unterschiedliche Geometrie, Freiheitsgrade und/oder Komponenten können beispielsweise von der Auswertvorrichtung in Abhängigkeit von vorgegebenen Randbedingungen, wie beispielsweise einer Größe und Aufteilung eines OP-Saals, ausgewählt werden oder durch einen Konstrukteur vorgegeben werden. Entsprechend wirken bei einem solchen anderen oder veränderten Tragarmsystem andere Momente und/oder Lagerkräfte, welche eine andere strukturelle Auslegung erfordern können. Die Auslegung kann beispielsweise jeweiligen Anforderungen an dessen maximale und/oder periodische Belastung entsprechen. Eine solche Berechnung kann beispielsweise mit Steifigkeitsrechnungen oder einem Simulationsmodell, beispielsweise mit einem CAD-Modell erfolgen. Ebenso können bei den strukturellen Anforderungen auch unterschiedliche Rahmenbedingungen an die Benutzung und damit Auslegung berücksichtigt werden. Beispielsweise kann die Auslegung für ein Krankenhaus mit größerer oder kleiner Auslastung optimiert werden und entsprechend erhöhter oder verringerter Benutzungsfrequenz. Ebenso kann/können auch als Rahmenbedingungen eine größere Lebensdauer und/oder längere Wartungsintervalle vorgegeben werden, wobei eine entsprechende Belastung für einen veränderten Zeitraum aus den gespeicherten Belastungsdaten extrapoliert werden kann. Auch bei einem unerwarteten oder frühzeitigen Versagen des Tragarmsystems, beispielsweise aufgrund von Materialermüdung, können mittels der Belastungsdaten veränderte strukturelle Anforderungen für ein Ersatztragarmsystem berechnet werden, um eine verbesserte Auslegung zu ermöglichen. Die Auswertvorrichtung kann beispielsweise als Computer ausgebildet sein.

Die Ergebnisse der Auswertung können ebenfalls von der Speichervorrichtung gespeichert werden, zusätzlich zu den Belastungsdaten. Die Auswertvorrichtung kann auch dazu ausgebildet sein, die Belastungsdaten unmittelbar - ohne vorherige Speicherung - auszuwerten. Die Speichervorrichtung kann dann auch die Ergebnisse dieser Auswertung als Belastungsdaten speichern. In diesem Fall können die Auswertungsergebnisse, wie die Wartungserfordernisse, auch als Belastungsdaten, insbesondere ausgewertete Belastungsdaten, angesehen werden.

Das Tragarmsystem umfasst ferner mehrere aneinander gelagerte bzw. angeordnete Tragarme, wobei die Belastungserfassungsvorrichtung dazu ausgebildet ist, für die jeweiligen Tragarme und/oder jeweilige Lagerungen die Belastung getrennt zu erfassen. Jeweilige beweglich aneinander gelagerte Tragarme sind vorzugsweise mittels jeweils dazwischen angeordneter und zugeordneter Lagerungen aneinandergelagert. Vorzugsweise ist die Speichervorrichtung dazu ausgebildet, getrennt und/oder den jeweiligen Komponenten des Tragarmsystems zugeordnet die entsprechenden Belastungsdaten zu speichern. Durch eine Vielzahl von Tragarmen kann das Tragarmsystem besonders viele Freiheitsgrade aufweisen, mehrere medizintechnische Geräte halten, einen besonders großen Bewegungsspielraum haben und/oder besonders gut an eine komplexe Raumgeometrie und weitere Randbedingungen, wie im Raum angeordnete weitere medizintechnische Geräte, angepasst sein.

In weiterer vorteilhafter Ausgestaltung des Tragarmsystems ist es vorgesehen, dass die Belastungserfassungseinrichtung dazu ausgebildet ist, jeweilige absolute Positionen der einzelnen Tragarme zu erfassen. Dadurch können auch stationäre Belastungen gut erfasst werden und die Genauigkeit der Daten kann besonders hoch sein. Insbesondere kann hierfür gegebenenfalls eine einmalige Kalibrierung jeweiliger Sensoren ausreichend sein.

In weiterer vorteilhafter Ausgestaltung des Tragarmsystems ist es vorgesehen, dass die Belastungserfassungseinrichtung dazu ausgebildet ist, jeweilige relative Positionen der einzelnen Tragarme zueinander zu erfassen. Dadurch kann die Belastungserfassungseinrichtung und deren jeweilige Sensoren besonders einfach und kostengünstig sein. Auch auf eine Kalibrierung könnte dann gegebenenfalls verzichtet werden. Dies ist besonders geeignet, um kostengünstig Belastungen durch Beschleunigungen zu erfassen.

In weiterer vorteilhafter Ausgestaltung des Tragarmsystems ist es vorgesehen, dass die Belastungserfassungseinrichtung dazu ausgebildet ist, jeweilige Bewegungen und/oder Beschleunigungen einzelner Tragarm zu erfassen. Dadurch können individuell auf einzelne Tragarme wirkende Belastungen besonders präzise erfasst werden.

In weiterer vorteilhafter Ausgestaltung des Tragarmsystems ist es vorgesehen, dass die Belastungserfassungseinrichtung dazu ausgebildet ist, jeweilige Kräfte, insbesondere jene, die auf einzelne Tragarme wirken, zu erfassen. Durch eine direkte Krafterfassung kann auf eine gegebenenfalls aufwendige Rückrechnung auf Belastungen verzichtet werden, wodurch das System weniger Rechenleistung benötigt und dessen Software einfach und kostengünstig ausgestaltet sein kann. Zudem können jeweilige erfasste externe Kräfte einfach zur Steuerung weiterer Komponenten genutzt werden, beispielsweise zum Ansteuern von Aktuatoren und/oder gegebenenfalls vorliegender Bremsvorrichtungen.

In weiterer vorteilhafter Ausgestaltung des Tragarmsystems ist es vorgesehen, dass die Belastungserfassungseinrichtung dazu ausgebildet ist, Vibrationen, insbesondere solche, welche einzelne Tragarme und/oder Lager erfahren, zu erfassen. Vibrationen können besonders relevant für Verschleiß sein, beispielsweise aufgrund von Materialermüdung und Lockern von Schraubverbindungen, und besonders gut Rückschlüsse auf Fehlgebrauch und Fehlbedienungen erlauben, beispielsweise ein Anschlagen an einem anderen Objekt beim Bewegen. Vibrationen können auch durch Kraft und/oder Beschleunigungsmessung mittelbar erfasst werden, wobei beispielsweise eine hohe zeitliche Auflösung der Messung sinnvoll sein könnte, welche auch als Abtastrate bezeichnet werden kann.

Die oben genannten Erfassungen können auch vorteilhaft miteinander kombiniert werden, insbesondere wenn für unterschiedliche Erfassungen die gleichen Sensoren genutzt werden können oder sollen. Dann kann das Tragarmsystem erschöpfend nützliche Daten zu dessen Gebrauch im täglichen Einsatz bei geringen Kosten bereitstellen.

In weiterer vorteilhafter Ausgestaltung des Tragarmsystems ist es vorgesehen, dass die Belastungserfassungseinrichtung wenigstens einen Sensor aufweist, insbesondere wenigstens einen einem Tragarm und/oder einer Lagerung zugeordneten Sensor. Die Sensoren ermöglichen eine genaue Erfassung individueller Belastungsdaten. Durch zugeordnete Sensoren können diese vorteilhaft angeordnet werden, beispielsweise am Ort jeweiliger größter erwarteter Belastungen oder nahe an fehlbedienungsanfälligen oder beschädigungsanfälligen Bereichen und/oder Teilen, wodurch die erfassten Sensordaten für die Belastung und/oder Wartungserfordernisse besonders aussagekräftig sind.

In weiterer vorteilhafter Ausgestaltung des Tragarmsystems ist es vorgesehen, dass der wenigstens eine Sensor als Kraftsensor, Beschleunigungssensor, Torsionssensor, Drehmomentssensor, Positionssensor, Beschleunigungssensor, Dehnungsmessstreifen, Anschlagsensor, GPS-Sensor, DGPS-Sensor, Magnetfeldsensor, Helligkeitssensor, Ultraschallsensor, Gyroskop, und/oder Drucksensor ausgebildet ist. Insbesondere können pro Tragarm und/oder pro Lagerung jeweils mehrere zugeordnete Sensoren unterschiedlichen Typs, beispielsweise Positionssensor und Beschleunigungssensor vorgesehen sein. Ein Sensor kann auch als eine Kombination mehrerer Typen ausgebildet sein, beispielsweise kann ein Dehnungsmessstreifen dazu angeordnet sein, auch eine Torsion zu erfassen. Der wenigstens eine Sensor kann auch mehrere Sensortypen vereinigen, bspw. Beschleunigungssensor als Bewegungs- und Kraftsensor, insbesondere durch eine mittelbare Erfassung, beispielsweise mittels Rückrechnung.

Der Kraftsensor ist besonders geeignet dazu, Belastungen direkt zu messen und auch stationäre Belastungen zu erfassen, beispielsweise auch zu schwere gehaltene medizintechnische Geräte von Übergewicht. Ein Beschleunigungssensor ist besonders gut dafür geeignet, Bewegungen und Anschläge zu erfassen und ist kostengünstig und leicht. Ein Torsionssensor erlaubt die Erfassung ungewöhnlicher Fehlgebrauchslasten, wie unzulässig exzentrisch gehaltene Geräte und ein versuchtes Erzwingen einer Bewegung über den Bewegungsspielraum des Tragarmsystems hinaus. Ein Drehmomentsensor ist besonders gut dafür geeignet, Belastungen jeweiliger Lager zu erfassen. Ein Positionssensor erleichtert es, ein Bewegungsprofil einzelner Tragarme zu erfassen und mit den gespeicherten Belastungsdaten zu visualisieren. Ein Dehnungsmessstreifen kann sehr frühzeitig und direkt eine Materialermüdung erkennen. Ein Anschlagsensor, beispielsweise als Knopf ausgebildet, kann besonders kostengünstig Fehlbedienungen erkennen. Ein GPS-Sensor oder DGPS-Sensor kann Positionsdaten einzelner Komponenten direkt erfassen, insbesondere unabhängig von einer Kalibrierung vor Ort. Zudem können so auch Standortdaten direkt miterfasst werden, um regionale Belastungsunterschied, z.B. je nach Land, erfassen zu können, und Techniker zur Wartung direkt zum Standort des jeweiligen Tragarmsystems zu leiten. Ein Magnetfeldsensor kann besonders kostengünstig sein und zudem passiv betrieben werden, z.B. ohne Stromversorgung und ohne die Notwendigkeit von gegebenenfalls unerwünschter elektromagnetischer Interferenz, z.B. beim Einsatz in der Nähe bildgebender Geräte wie CTs. Ein einfaches und kostengünstiges Beispiel ist ein Kompass. Gleiches gilt für einen Helligkeitssensor, welcher zudem Distanzmessungen und damit drohende Kollisionen mit anderen Objekten, z.B. Gegenständen, detektieren kann. Ein Ultraschallsensor kann rechtzeitig eine drohende Kollision mit anderen Gegenständen erfassen, um dann beispielsweise vor Zusammenstoß eine Bremse zu aktivieren. Dies kann dann auch zur Erfassung jeweiliger Bewegungen genutzt werden. Ein Gyroskop erlaubt eine besonders präzise Lagebestimmung und kann auch Daten zur Steuerung einer Roboteroperationsvorrichtung liefern. Drucksensoren sind Kraftsensoren, welche eine flächige Belastung erfassen können und damit zusätzliche Belastungsinformationen ermöglichen.

In weiterer vorteilhafter Ausgestaltung des Tragarmsystems ist es vorgesehen, dass die Belastungserfassungseinrichtung, insbesondere deren jeweiligen Sensoren, kabelgebunden oder kabellos zur Datenübertragung mit der Speichervorrichtung verbunden ist bzw. sind. Eine kabelgebundene Datenverbindung ist besonders robust und kostengünstig und braucht zudem wenig Energie. Durch eine kabellose Datenübertragung ist die Gestaltung und Bewegung des Tragarmsystems bzw. der Tragarme besonders frei und das System besonders leicht. Insbesondere kann die Belastungserfassungseinrichtung dazu ausgebildet sein, mit dem Internet und/oder einem Mobilfunknetz und/oder Telefonnetz Belastungsdaten an die Speichervorrichtung zu übertragen. Damit ist die Speichervorrichtung nicht mehr an den Ort der weiteren Komponenten des Tragarmsystems, insbesondere der jeweiligen Tragarme, gebunden. Dadurch kann die weitere Auswertung dezentral und unabhängig erfolgen. Für die Datenübertragung kann/können die Belastungserfassungseinrichtung und/oder die jeweiligen Sensoren einen Zwischenspeicher aufweisen. Dieser Speicher kann auch zur Sicherstellung von Informationsredundanz dienen, insbesondere bei Ausfall der Datenübertragung, und auch als Teil der Speichervorrichtung angesehen werden. Die Daten können beispielweise kontinuierlich oder intermittierend übertragen werden.

In weiterer vorteilhafter Ausgestaltung des Tragarmsystems ist es vorgesehen, dass die Speichervorrichtung als zentraler Server ausgebildet ist, insbesondere zur Speicherung erfasster Belastungsdaten von Belastungserfassungseinrichtungen von mehreren Tragarmsystemen. Die Tragarmsysteme können identisch oder unterschiedlich ausgebildet sein. So können beispielsweise jeweilige Belastungsdaten zentral beim Hersteller oder Wartungspersonal gesammelt und gespeichert werden. Auch die Auswertvorrichtung kann als zentraler Server ausgebildet sein, wobei der zentrale Server dann sowohl als Speichervorrichtung als auch als Auswertvorrichtung ausgebildet sein kann. So kann das System besonders kostengünstig ausgestaltet sein und eine große Menge an Belastungsdaten sammeln. Vorzugsweise werden die Belastungsdaten dann per Mobilfunk und/oder Telefonnetz übertragen, beispielsweise über das Internet. So ist die Datenübertragung zeitnah und kostengünstig. Alternativ kann auch intermittierende physische Übertragung, beispielsweise mit einem Datenträger oder Auslesen vor Ort an der Belastungserfassungsvorrichtung, erfolgen. Dadurch könnte beispielsweise auf einen Anschluss an das Mobilfunknetz oder Festnetz verzichtet werden.

Ein weiterer Aspekt der Erfindung betrifft ein Tragarmbelastungserfassungssystem mit mehreren Tragarmsystem gemäß dem ersten Aspekt der Erfindung und einer Speichervorrichtung zum zentralen Speichern der Belastungsdaten aller Tragarmsysteme und/oder einer Auswertvorrichtung zum zentralen Auswerten der Belastungsdaten aller im Einsatz befindlichen Tragarmsysteme eines oder mehrerer Nutzer/s. Dadurch kann/können auf eine jeweils zugeordnete Speichervorrichtung und/oder eine jeweils zugeordnete Auswertvorrichtung bei mehreren Tragarmsystemen verzichtet werden. Ein solches Tragarmbelastungserfassungssystem wird besonders kostengünstig sein, da die Belastungsdaten einfach und sicher zentral gespeichert und verarbeitet bzw. ausgewertet werden können.

Ein weiterer Aspekt der Erfindung betrifft ein Verfahren zum Betreiben eines erfindungsgemäßen Tragarmsystems zum bewegbaren Halten wenigstens eines medizinischen Geräts nach Anspruch 12. Das Verfahren umfasst den Schritt des Benutzens des Tragarmsystems zum Halten eines medizinischen Geräts. Das Benutzen betrifft auch ein Bewegen des gehaltenen Geräts und/oder einen Einsatz während einer medizinischen Behandlung eines Patienten mit dem Gerät. Hierbei wird der bewegbar gelagerte Tragarm des Tragarmsystems mit wenigstens einem daran gehaltenen medizinischen Gerät bewegt. Das Bewegen kann manuell bewirkt oder ausgelöst werden und/oder mittels eines Aktuators.

Weiterhin weist das Verfahren zum Betreiben des Tragarmsystems den Schritt des Erfassens jeweiliger Belastungsdaten des Tragarmsystems durch dessen Benutzung, insbesondere durch ein Bewegen des Tragarms auf. Als weiterer Schritt werden die erfassten Belastungsdaten gespeichert. Das Erfassen kann dabei mittels einer Belastungserfassungsvorrichtung des Tragarmsystems erfolgen und/oder das Speichern mittels einer Speichervorrichtung des Tragarmsystems. Insbesondere kann dies automatisch erfolgen, beispielsweise kann eine erfasste Bewegung ein automatisches Speichern jeweiliger korrespondierender Belastungsdaten bewirken.

Durch ein solches Betreiben kann das Tragarmsystem besonders bedarfsgerecht gewartet werden. Zudem können nachfolgende Systemgenerationen anhand der Belastungsdaten vorteilhaft bedarfsgerecht gestaltet werden, da z.B. individuelle strukturelle Anforderungen durch die gespeicherten Belastungsdaten sehr genau bewertet und für den zukünftigen Einsatz vorhergesagt werden können, wodurch diese besonders kostengünstig und wartungsarm sein können.

Das Verfahren gemäß diesem Erfindungsaspekt ist zum Betreiben eines Tragarmsystems gemäß dem ersten Erfindungsaspekt ausgebildet und geeignet. Die sich aus dem Tragarmsystem gemäß dem ersten Erfindungsaspekt ergebenden Merkmale und Vorteile sind den Beschreibungen des ersten Erfindungsaspekts zu entnehmen, wobei vorteilhafte Ausgestaltungen des ersten Erfindungsaspekts als vorteilhafte Ausgestaltungen des weiteren Erfindungsaspekts und umgekehrt anzusehen sind.

In weiterer vorteilhafter Ausgestaltung des Verfahrens zum Betreiben des Tragarmsystems ist es vorgesehen, dass jeweilige Wartungserfordernisse für das Tragarmsystem in Abhängigkeit von den gespeicherten Belastungsdaten bestimmt werden und alternativ oder zusätzlich strukturelle Anforderungen an die Auslegung eines weiteren Tragarmsystems vorzugsweise für den gleichen Einsatz in Abhängigkeit von den gespeicherten Belastungsdaten bestimmt werden. Dies kann mittels einer Auswertvorrichtung des Tragarmsystems erfolgen. Insbesondere kann dies auch in Abhängigkeit von einem gespeicherten zeitlichen Verlauf der Belastungsdaten bzw. der Belastung erfolgen. Die Auswertung kann auch unmittelbar ohne vorherige Speicherung erfolgen und/oder deren Ergebnisse als ausgewertete Belastungsdaten gespeichert werden, insbesondere mittels der Speichervorrichtung.

Ein noch weiterer Aspekt der Erfindung betrifft ein Verfahren zum Gestalten eines Tragarmsystems zum bewegbaren Halten wenigstens eines medizinischen Geräts mittels eines bewegbar gelagerten Tragarms nach Anspruch 14. Das Verfahren weist das Aufstellen eines Testtragarmsystems, welches auch als Testsystem bezeichnet wird, mit wenigstens einem bewegbar gelagerten Tragarm an dem gewünschten Einsatzort und/oder zur Nutzung für denselben Einsatzzweck wie das zu entwerfende Tragarmsystem, auf. Die festzulegenden Strukturmerkmale des Tragarmsystems können beispielsweise ausgewählt sein aus der Gruppe bestehend aus Tragarmlänge, Wandstärke, Materialauswahl der jeweiligen Strukturkomponenten und/oder Lager(typ). Der Einsatzort kann gegebenenfalls auch simuliert werden, um eine Testphase auch beim Hersteller durchführen zu können. Vorzugsweise wird das Testsystem aber beim Kunden des zu gestaltenden Tragarmsystems aufgestellt, insbesondere in dem OP-Saal, in welchem das fertige System auch zum Einsatz kommen soll; vorteilhafterweise zur Nutzung unter anwendungstypischen Bedingungen und medizinischen Behandlungen. Ein weiterer Schritt betrifft die Nutzung des Testtragarmsystems, insbesondere zum Datensammeln. Während des Benutzungszeitraums werden jeweilige Belastungsdaten des Testtragarmsystems bei dessen Benutzung erfasst, insbesondere ein Bewegen dessen Tragarme. Das Testtragarmsystem kann also unter realen Einsatzbedingungen verwendet werden, um Belastungsdaten zu erfassen. Die erfassten Belastungsdaten des Testtragarmsystems werden gespeichert. Das tatsächlich für die Dauernutzung schließlich bereitgestellte Tragarmsystem wird dann in Abhängigkeit von den gespeicherten Belastungsdaten des Testtragarmsystems ausgestaltet. Dadurch können die Anforderungen, insbesondere strukturellen Anforderungen, ermittelt werden. Das Gestalten kann insbesondere einen oder mehrere Entwürfe des Tragarmsystems umfassen, dessen grundsätzliche Konzeptionierung, dessen Auslegung und/oder Bestimmung von Anforderungen, z.B. hinsichtlich struktureller Belastungsanforderungen, Freiheitsgrade, Größe, Bewegungsreichweite, und dessen eigentliche Konstruktion, z.B. Robustheit der zu verbauenden Komponenten (beispielsweise Lager oder Gelenke) oder die Materialwahl. Das zu gestaltende Tragarmsystem kann so individuell bedarfsgerecht und damit kostenoptimiert ausgestaltet sein, insbesondere für einen spezifischen Einsatzort und Einsatzbedingungen und/oder einen bestimmten Kunden und dessen Anforderungen und Nutzung optimiert werden.

Das Testtragarmsystem ist als Tragarmsystem gemäß dem ersten Aspekt der Erfindung ausgebildet. Es kann mit dem Verfahren gemäß dem weiteren Aspekt der Erfindung betrieben werden. Das Verfahren gemäß diesem Aspekt der Erfindung kann auch eine Verwendung des Tragarmsystems gemäß dem ersten Aspekt der Erfindung sowie des Verfahrens gemäß dem weiteren Erfindungsaspekt betreffen. Die sich aus dem Tragarmsystem gemäß dem ersten Erfindungsaspekt und dem Verfahren gemäß dem weiteren Erfindungsaspekt ergebenden Merkmale und Vorteile sind den Beschreibungen des ersten bzw. weiteren Erfindungsaspekts zu entnehmen, wobei vorteilhafte Ausgestaltungen des ersten bzw. weiteren Erfindungsaspekts als vorteilhafte Ausgestaltungen des noch weiteren Erfindungsaspekts und umgekehrt anzusehen sind.

Das zu gestaltende Tragarmsystem kann auch ein Tragarmsystem gemäß dem ersten Erfindungsaspekt sein. Alternativ kann das zu gestaltende Tragarmsystem auch ein herkömmliches System ohne Belastungsdatenerfassung sein. Das Testsystem und das zu gestaltende Tragarmsystem können strukturell ähnlich oder identisch sein oder auch unterschiedlich sein. Beispielsweise kann das Testtragarmsystem eine größere Anzahl an Freiheitsgraden und einen größeren Bewegungsspielraum haben und zudem für sehr hohe Lasten ausgelegt sein, damit es alle potentiellen Kundenanforderungen beim Testeinsatz abbilden kann. In Abhängigkeit von den erfassten und gespeicherten Belastungsdaten kann dann bestimmt werden, wieviele Freiheitsgrade und welcher Bewegungsspielraum beim - mittels der erhobenen Daten - zu gestaltenden Tragarmsystem tatsächlich in diesem Anwendungsfall notwendig oder ökonomisch sinnvoll sind und/oder welche Lasten dieses wirklich tragen muss, um das Tragarmsystem dann entsprechend optimiert für den Kunden auszugestalten.

Weitere Merkmale der Erfindung ergeben sich in den Ansprüchen, dem Ausführungsbeispiel sowie anhand der Zeichnungen. Die vorstehend in der Beschreibung genannten Merkmale und Merkmalskombinationen sowie die nachfolgend in den Ausführungsbeispielen genannten Merkmale und Merkmalskombinationen sind nicht nur in der jeweils angegebenen Kombination, sondern auch in anderen Kombinationen verwendbar.

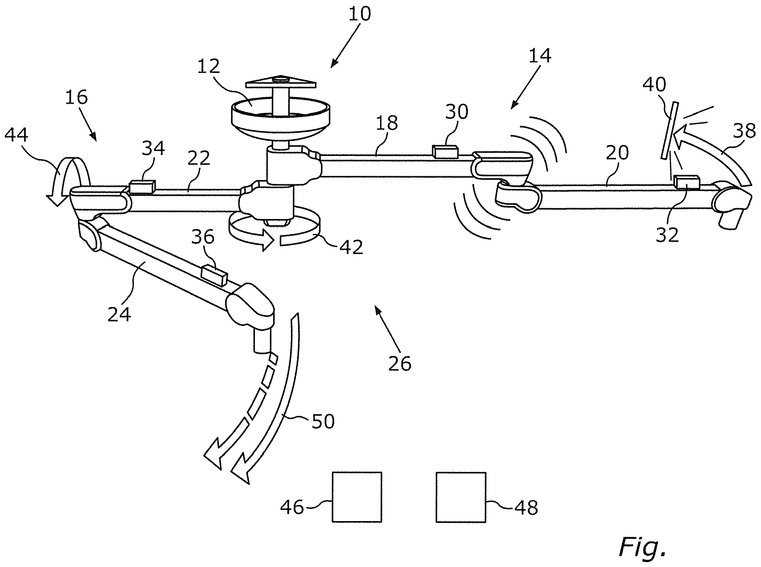

Dabei zeigt die einzige Figur ein Tragarmsystem mit einer Belastungserfassungsvorrichtung.

Die Figur veranschaulicht schematisch ein Tragarmsystem 10, welches fest an der Decke eines OP-Saals mittels der Verankerung 12 angebracht ist. An der Verankerung 12 sind zwei Zweige 14, 16 zum Halten jeweils eines medizintechnischen Geräts drehbar angeordnet, beispielsweise mittels jeweils eines Kugellagers. Der erste Zweig 14 weist einen ersten, unmittelbar an der Verankerung 12 schwenkbar gelagerten Tragarm 18 auf. Die Schwenkbewegung ist durch Pfeil 42 veranschaulicht. An diesem ersten Tragarm 18 ist ein zweiter Tragarm 20 verbunden, welcher mit dem ersten Tragarm 18 mittels ebenfalls einer Lagerung zum relativen Verschwenken der zwei Tragarme 18, 20 verbunden ist. An dem dem ersten Tragarm 18 abgewandten Ende des zweiten Tragarms 20 ist ein nicht dargestelltes medizintechnisches Gerät befestigt, vorliegend mittels eines Kugelgelenks. Bei dem Gerät kann es sich beispielsweise um eine OP-Lampe handeln. Der erste Zweig 14 hat somit zwei rotatorische Freiheitsgrade zum Bewegen der Tragarm 18, 20 und das medizintechnische Gerät ist aufgrund des Kugelgelenks mit weiteren Freiheitsgraden bewegbar. Damit kann ein Operateur die Beleuchtung des OP-Tisches sehr frei und präzise einstellen.

Der zweite Zweig 16 weist eine zum ersten Zweig 14 ähnliche Bauweise auf. Der zweite Zweig 16 umfasst den dritten Tragarm 22 und den vierten Tragarm 24. Der dritte Tragarm 22 ist unmittelbar unter dem ersten Tragarm 18 ebenfalls schwenkbar an der Verankerung 12 gelagert. Die Schwenkbewegung ist ebenfalls durch Pfeil 42 veranschaulicht. An dem dritten Tragarm 22 ist der vierte Tragarm 24 ebenfalls verschwenkbar gelagert. An dem dem dritten Tragarm 22 abgewandten Ende des vierten Tragarms 24 ist ein nicht dargestelltes medizintechnisches Gerät befestigt, vorliegend mittels eines Kippgelenks. Damit kann eine Neigung des medizinischen Geräts gegenüber der Horizontalen einfach und bequem eingestellt werden. An dem zweiten Zweig 16 kann so beispielsweise als Gerät eine Halterung für das OP-Besteck gehalten sein.

Weiterhin kann eine, mehrere oder alle der Lagerungen eine Bremse einer Bremsvorrichtung aufweisen, welche vorliegend nicht dargestellt ist. Damit kann eine relative Bewegung der Tragarm 18, 20, 22, 24 zueinander und zu der Verankerung 12 sowie ein Bewegen der beiden medizinischen Geräte gehemmt oder verhindert werden. Mit der Bremse kann also ein Verbleib des Tragarmsystems 10 in einer eingestellten Position sichergestellt werden.

Offensichtlich unterliegt das Tragarmsystem 10 sowohl durch Bewegen der einzelnen Komponenten als auch durch Öffnen und Schließen der Bremsen und damit jeweils Aktivieren und Deaktivieren der Bewegungshemmung einem Verschleiß, welcher Wartungen und/oder Inspektionen notwendig macht. Die Anforderungen an ein Tragarmsystem für medizintechnische Geräte sind dabei besonders hoch, da eine Fehlfunktion, wie eine Blockade oder ein Tragarmbruch, den Patienten im Operationssaal gefährden könnten bzw. den Operationsverlauf beeinträchtigen könnten. Zudem kann ein Verschleiß auch die Hygiene eines OP-Saals negativ beeinflussen, beispielsweise durch herabfallenden Bremsstaub. Zudem kann das Tragarmsystem 10 bereits dadurch Fehlbedienungen erfahren, beispielsweise indem es gegen andere Gegenstände im OP-Saal versehentlich geschlagen wird. Auch Fehlgebrauchslasten, wie ein unzulässiges Abstützen eines Operateurs an einem der Zweige 14, 16, können das Tragarmsystem 10 beschädigen und/oder eine Wartung erforderlich machen.

Aus diesem Grund sind herkömmliche Tragarmsysteme mit hohen Sicherheitsmargen strukturell ausgelegt und müssen häufig und aufwendig inspiziert und gewartet werden. Die hohen Sicherheitsmargen verlangen teure und schwerfällige Systeme. Die aufwendige Wartung und Inspektion resultieren in hohen laufenden Kosten zum Unterhalt des Tragarmsystems.

Vorliegend weist das Tragarmsystem jedoch eine Belastungserfassungseinrichtung 26 auf, mittels welcher jeweilige Belastungsdaten des Tragarmsystems 10 bei dessen Benutzung, insbesondere durch Bewegen und/oder Belasten der Zweige 14, 16, erfasst werden können.

Dazu umfasst die Belastungserfassungseinrichtung 26 einen ersten Sensor 30, welcher als Beschleunigungssensor ausgebildet ist und nahe zu der Verbindung mit dem zweiten Tragarm 20 an dem ersten Tragarm 18 bzw. nahe an deren Lagerung miteinander angeordnet ist. Mittels des ersten Sensors 30 kann derart eine Bewegung des ersten Tragarms 18 erfasst werden. Insbesondere kann der erste Sensor 30 gegebenenfalls hochsensitiv ausgestaltet sein, so dass er auch Vibrationen der Lagerung zwischen dem ersten Tragarm 18 und dem zweiten Tragarm 20 messen kann. Dazu kann der Sensor 30 eine hohe Abtastrate haben und dazu ausgebildet sein, auch kurze und/oder sehr geringfügige Beschleunigungen zu erfassen. Durch die Erfassung kann sowohl auf eine Belastung dieser Lagerung als auch des ersten Tragarms 18 und dessen Lagerung an der Verankerung 12 rückgeschlossen werden.

Diese Erfassung und der Rückschluss auf die Belastung kann durch den zweiten Sensor 32 an dem zweiten Tragarm 20 weiter verbessert werden. Dieser kann beispielsweise ebenfalls als Beschleunigungssensor ausgebildet sein, womit die Bewegung der beiden Tragarme 18, 20 getrennt voneinander erfasst werden kann. Zudem kann durch den zweiten Sensor 32 eine Fehlbedienung erkannt werden, beispielsweise ein Anschlagen an einem anderen Objekt oder einer Wand. Dies ist in der Figur durch den Pfeil 38 und das Objekt 40 veranschaulicht.

Analog weist auch der zweite Zweig 16 einen dritten Sensor 34 und einen vierten Sensor 36 auf. Der dritte Sensor 34 kann beispielsweise auch dazu ausgebildet sein, eine Torsion an der Lagerung zwischen den beiden Tragarmen 22, 24 zu erfassen bzw. eine Neigung, wie es durch Pfeil 44 veranschaulicht ist. Dadurch sind Belastungsdaten hinsichtlich des Gewichts des an dem zweiten Zweig 16 gehaltenen Geräts und auch Belastungsdaten hinsichtlich eines missbräuchlichen Abstützens verfügbar.

Der vierte Sensor 34 kann dazu ausgebildet sein, eine Stellung des Gelenks zum Halten des medizinischen Geräts an dem zweiten Zweig 16 und damit eine Neigung gegenüber der Horizontalen zu erfassen, was vorliegend durch Pfeil 50 veranschaulicht ist. Dadurch kann der Sensor 34 auch als Signalgeber an einen Aktuator dienen, der das medizinische Gerät am zweiten Zweig 16 automatisch in der Horizontalen hält. Insgesamt können die jeweiligen Sensoren 30, 32, 34, 36 auch dazu ausgebildet sein, zu erfassen, ob ein Benutzer versucht, einen Teil des Tragarmsystems 10 zu bewegen. Dann kann bei einer solchen Erfassung automatisch eine Hemmung bzw. Blockierung einer solchen Bewegung durch die Bremsvorrichtung deaktiviert werden, wodurch das Tragarmsystem 10 besonders komfortabel zu benutzen ist. Nach Beendigung der Bewegung kann die Bremsvorrichtung dann automatisch wieder aktiviert werden.

Die Sensoren 30, 32, 34, 36 können beispielsweise auch als Helligkeitssensoren oder Magnetsensoren ausgebildet sein und Lage und Bewegung auch mittels Veränderung der Helligkeit oder eines Magnetfelds erfassen. Auch das Vorsehen eines Kompasses als Sensor ist möglich, der dann eine Schwenkbewegung in der Horizontalen kostengünstig erfassen kann.

Die Belastungsdaten werden von der Belastungserfassungsvorrichtung 26 an die Speichervorrichtung 46 übertragen. Die Speichervorrichtung 46 kann die Rohsensordaten speichern oder diese auch vorher bereits verrechnen, also etwa in Belastungen umrechnen, oder beides. Eine Übertragung kann beispielsweise kabellos oder kabelgebunden erfolgen. Durch die Speichervorrichtung 26 stehen die Belastungsdaten zur Verfügung, um beispielsweise die Notwendigkeit einer Wartung und/oder eines Austausches von Teilen des Tragarmsystems 10 zu ermitteln. So kann die Wartung und Austausch bedarfsgerecht nach der tatsächlichen individuellen Benutzung und Belastung des Tragarmsystems 10 erfolgen, wodurch der Unterhalt besonders kostengünstig ist. So können beispielsweise auch Krankenhäuser das Tragarmsystem 10 mit nur gelegentlicher Tragarmsystemnutzung verwenden, dessen geringe Auslastung und Benutzung anderenfalls eine standardisierte und in gleichmäßigen Intervallen unabhängig von der tatsächlichen Belastung erfolgende Wartung und Austausch nicht rechtfertigen würde. In solchen Fällen unterbliebe gegebenenfalls die Anschaffung wegen regelmäßiger (aber unnötiger) Wartungs- oder Inspektionskosten für herkömmliche Tragarmsysteme. Zudem können die Belastungsdaten bei der Auslegung und Konstruktion von Weiterentwicklungen oder anderen Tragarmsystemen verwendet werden, um diese weiter zu optimieren.

Das Tragarmsystem 10 umfasst für die oben genannten Zwecke noch eine Auswertvorrichtung 48, mittels welcher in Abhängigkeit von den gespeicherten Belastungsdaten jeweilige Wartungserfordernisse für das Tragarmsystem 10 automatisch bestimmt werden und welches auch in Abhängigkeit von den gespeicherten Belastungsdaten strukturelle Anforderungen an die Auslegung eines weiteren Tragarmsystems für den gleichen Einsatz oder andere Einsätze bestimmen kann, insbesondere mittels Extrapolation. Die Auswertvorrichtung 48 kann mit der Speichervorrichtung 46 zur Datenübertragung beispielsweise über das Internet verbunden sein, sodass die Auswertvorrichtung 48 beispielsweise nicht im OP-Saal untergebracht werden muss, sondern beispielsweise durch einen Rechner bei dem Hersteller des Tragarmsystems 10 bereitgestellt werden kann. Ebenso kann die Speichervorrichtung 46 auch die Sensordaten über das Internet von den Sensoren 30, 32, 34, 36 erhalten, welche dafür einen Mobilfunkchip aufweisen können und/oder mit einer gemeinsamen Übertragungsvorrichtung verbunden sein können. Damit kann dann sowohl die Speichervorrichtung 46 als auch die Auswertvorrichtung 48 gemeinsam durch einen zentralen Server des Herstellers gebildet werden. Insbesondere können die Speichervorrichtung 46 und/oder die Auswertvorrichtung 48 dazu ausgebildet sein, die Belastungsdaten von mehreren Tragarmsystemen 10 mit Belastungsdatenerfassung zu speichern und/oder auszuwerten, sodass eine besonders große Datenbasis erzeugt werden kann.

### Bezugszeichenliste

- 10: Tragarmsystem
- 12: Verankerung
- 14: erster Zweig
- 16: zweiter Zweig
- 18: erster Tragarm
- 20: zweiter Tragarm
- 22: dritter Tragarm
- 24: vierter Tragarm
- 26: Belastungserfassungsvorrichtung
- 30: erster Sensor
- 32: zweiter Sensor
- 34: dritter Sensor
- 36: vierter Sensor
- 38: Pfeil
- 40: Objekt
- 42: Pfeil
- 44: Pfeil
- 46: Speichervorrichtung
- 48: Auswertvorrichtung

## Patentansprüche

1. Tragarmsystem (10) zum bewegbaren Halten wenigstens eines medizinischen Geräts, umfassend wenigstens einen bewegbar gelagerten Tragarm (18, 20, 22, 24), welcher zum Halten des wenigstens einen medizinischen Geräts ausgebildet ist, wenigstens eine Belastungserfassungsvorrichtung (26), welche zum Erfassen jeweiliger Belastungsdaten des Tragarmsystems (10) durch dessen Benutzung ausgebildet ist, wobei das Tragarmsystem (10) mehrere aneinander gelagerte Tragarme (18, 20, 22, 24) umfasst, wobei die Belastungserfassungsvorrichtung (26) dazu ausgebildet ist, für die jeweiligen Tragarme (18, 20, 22, 24) und/oder deren jeweilige Lagerungen die Belastung getrennt zu erfassen, und wenigstens eine Speichervorrichtung (46), welche zum Speichern der erfassten Belastungsdaten ausgebildet ist.

2. Tragarmsystem (10) nach Anspruch 1, wobei die wenigstens eine Belastungserfassungsvorrichtung (26) zum Erfassen jeweiliger Belastungsdaten des Tragarmsystems (10) durch ein Bewegen des Tragarms (18, 20, 22, 24) ausgebildet ist.

3. Tragarmsystem (10) nach Anspruch 1 oder 2, wobei das Tragarmsystem (10) ferner einer Auswertvorrichtung (48) umfasst, welche dazu ausgebildet ist, in Abhängigkeit von den gespeicherten Belastungsdaten jeweilige Wartungserfordernisse für das Tragarmsystem (10) zu bestimmen und/oder in Abhängigkeit von den gespeicherten Belastungsdaten strukturelle Anforderungen an die Auslegung, insbesondere ausgewählt aus der Gruppe bestehend aus Lagertyp, Materialauswahl der Strukturkomponenten, Wandstärke und Lagerstruktur eines weiteren Tragarmsystems für den gleichen Einsatz zu bestimmen.

4. Tragarmsystem (10) nach einem der vorhergehenden Ansprüche, wobei die Belastungserfassungseinrichtung (26) dazu ausgebildet ist, jeweilige absolute Positionen jeweiliger Tragarme (18, 20, 22, 24) zu erfassen, jeweilige relative Positionen jeweiliger Tragarme (18, 20, 22, 24) zueinander zu erfassen, jeweilige Bewegungen und/oder
Beschleunigungen jeweiliger Tragarme (18, 20, 22, 24) zu erfassen, jeweilige Kräfte, insbesondere welche auf jeweilige Tragarme (18, 20, 22, 24) wirken, zu erfassen, und/oder jeweilige Vibrationen, insbesondere welche jeweilige Tragarme (18, 20, 22, 24) und/oder Lager erfahren, zu erfassen.

5. Tragarmsystem (10) nach einem der vorhergehenden Ansprüche, wobei die Belastungserfassungseinrichtung (26) wenigstens einen Sensor (30, 32, 34, 36) aufweist.

6. Tragarmsystem (10) nach dem vorhergehenden Anspruch, wobei die Belastungserfassungsvorrichtung (26) wenigstens einen einem Tragarm (18, 20, 22, 24) und/oder einer Lagerung zugeordneten Sensor (30, 32, 34, 36) aufweist.

7. Tragarmsystem (10) nach einem der vorhergehenden Ansprüche 5 oder 6, wobei der wenigstens eine Sensor (30, 32, 34, 36) als Kraftsensor, Beschleunigungssensor, Torsionssensor, Drehmomentsensor, Positionssensor, Dehnungsmessstreifen, Anschlagsensor, GPS-Sensor, DGPS-Sensor, Magnetfeldsensor, Helligkeitssensor, Ultraschallsensor, Gyroskop, und/oder Drucksensor ausgebildet ist.

8. Tragarmsystem (10) nach einem der vorhergehenden Ansprüche, wobei die Belastungserfassungseinrichtung (26), insbesondere deren jeweiligen Sensoren (30, 32, 34, 36), kabelgebunden oder kabellos zur Datenübertragung mit der Speichervorrichtung (46) verbunden ist bzw. sind.

9. Tragarmsystem (10) nach einem der vorhergehenden Ansprüche, wobei die Speichervorrichtung (46) als zentraler Server ausgebildet ist.

10. Tragarmsystem (10) nach Anspruch 9, wobei die Speichervorrichtung (46) zur Speicherung erfasster Belastungsdaten jeweiliger Belastungserfassungseinrichtungen (26) von mehreren Tragarmsystemen (10) ausgebildet ist.

11. Tragarmbelastungserfassungssystem mit mehreren Tragarmsystemen (10) nach einem der vorhergehenden Ansprüche und einer Speichervorrichtung (46) zum zentralen Speichern der Belastungsdaten aller Tragarmsysteme (10) und/oder einer Auswertvorrichtung (48) zum zentralen Auswerten der Belastungsdaten aller Tragarmsysteme (10).

12. Verfahren zum Betreiben eines Tragarmsystems (10) nach einem der Ansprüche 1 bis 10 zum bewegbaren Halten wenigstens eines medizinischen Geräts, wenigstens die folgenden Schritte aufweisend:
- Bewegen eines bewegbar gelagerten Tragarms (18, 20, 22, 24) des Tragarmsystems (10) mit wenigstens einem daran gehaltenen medizinischen Gerät;
- Erfassen jeweiliger Belastungsdaten des Tragarmsystems (10) durch dessen Benutzung, insbesondere durch ein Bewegen des Tragarms (18, 20, 22, 24); und
- Speichern der erfassten Belastungsdaten.

13. Verfahren nach Anspruch 12, ferner wenigstens die folgenden Schritte aufweisend:
- Bestimmen jeweiliger Wartungserfordernisse für das Tragarmsystem (10) in Abhängigkeit von den gespeicherten Belastungsdaten;
und/oder
- Bestimmen struktureller Anforderungen an die Auslegung eines weiteren Tragarmsystem in Abhängigkeit von den gespeicherten Belastungsdaten.

14. Verfahren zum Gestalten eines Tragarmsystems (10) zum bewegbaren Halten wenigstens eines medizinischen Geräts mittels eines bewegbar gelagerten Tragarms (18, 20, 22, 24), wenigstens die folgenden Schritte aufweisend:
- Aufstellen eines Testtragarmsystems nach einem der Ansprüche 1 bis 10 mit wenigstens einem bewegbar gelagerten Tragarm an dem gewünschten Einsatzort und/oder zur Nutzung für denselben Einsatzzweck wie das zu entwerfende Tragarmsystem;
- Benutzung des Testtragarmsystems;
- Erfassen jeweiliger Belastungsdaten des Testtragarmsystems bei dessen Benutzung, insbesondere durch ein Bewegen dessen Tragarms;
- Speichern der erfassten Belastungsdaten des Testtragarmsystems; und
- Gestalten des Tragarmsystems (10) in Abhängigkeit von den gespeicherten Belastungsdaten des Testtragarmsystems.

## Claims

1. Support arm system (10) for movably holding at least one medical device, comprising
at least one movably supported support arm (18, 20, 22, 24) which is configured to hold at least one medical device,
at least one load detection device (26) which is configurged to detect respective load data of the support arm system (10) during usage thereof, wherein the support arm system (10) comprises a plurality of support arms (18, 20, 22, 24) which are supported adjacent to one another, wherein the load detection device (26) is configured to individually detect the load for the respective support arms (18, 20, 22, 24), and/or the respective bearings.
and
at least one storage device (46) which is configured for storing the detected load data.

2. Support arm system (10) according to claim 1, wherein the at least one load detection device (26) is configured for detecting respective load data of the support arm system (10) during moving the support arm (18, 20, 22, 24),

3. Support arm system (10) according to claim 1 or 2, wherein
the support arm system (10) further comprises an evaluation device (48) which is configured to determine the respective maintenance requirements for the support arm system (10) depending on the stored load data, and/or to determine the structural requirements for a design of a further support arm system for the same usage depending on the stored load data, in particular selected from the group consisting of a type of bearing, material selection of the structural components, wall thickness, and bearing structure.

4. Support arm system (10) according to one of the preceding claims, wherein
the load detection device (26) is configured to
detect respective absolute positions of the respective support arms (18, 20, 22, 24),
detect respective positions of the respective support arms (18, 20, 22, 24) relative to each other,
detect respective movements and/or accelerations of the respective support arms (18, 20, 22, 24),
detect respective forces, in particular those acting on the respective support arms (18, 20, 22, 24), and/or
detect respective vibrations, in particular those which the respective support arms (18, 20, 22, 24) and/or bearings are subjected.

5. Support arm system (10) according to one of the preceding claims, wherein
the load detection device (26) comprises at least one sensor (30, 32, 34, 36).

6. Support arm system (10) according to the preceding claim, wherein the load detection device (26) comprises at least one sensor (30, 32, 34, 36) assigned to a support arm (18, 20, 22, 24) and/or to a bearing.

7. Support arm system (10) according to one of the preceding claims 5 or 6, wherein the at least one sensor (30, 32, 34, 36) is configured as a force sensor, acceleration sensor, torsion sensor, torque sensor, position sensor, strain gauge, abutment sensor, GPS sensor, DGPS sensor, magnetic field sensor, brightness sensor, ultrasonic sensor, gyroscope, and/or pressure sensor.

8. Support arm system (10) according to one of the preceding claims, wherein
the load detection device (26), in particular the respective sensors (30, 32, 34, 36) thereof, is/are connected by wire or wireless to the storage device (46) for data transmission.

9. Support arm system (10) according to one of the preceding claims, wherein
the storage device (46) is configured as a centralized server.

10. Support arm system (10) according to claim 9, wherein the storage device (46) is configured for storing detected load data of the respective load detection devices (26) from plurality of support arm systems (10).

11. Support arm load detection system comprising a plurality of support arm systems (10) according to one of the preceding claims, and a storage device (46) for storing the load data of all support arm systems (10) in a centralized way, and/or an evaluation device (48) for a centralized evaluation of load data of all support arm systems (10).

12. Method for operating a support arm system (10) according to one of claims 1 to 10 for moveably holding at least one medical device, comprising at least the following steps:
- moving of a movably supported support arm (18, 20, 22, 24) of the support arm system (10) including at least one medical device which is supported thereon;
- detecting respective load data of the support arm system (10) based on the use thereof, in particular by moving the support arm (18, 20, 22, 24); and
- storing the detected load data.

13. Method according to claim 12, further comprising at least the following steps:
- determining the respective maintenance requirements for the support arm system (10) depending on the stored load data;
and/or
- determining structural requirements regarding the design of a further support arm system depending on the stored load data.

14. Method for designing a support arm system (10) for movably holding at least one medical device by means of a movably supported support arm (18, 20, 22, 24), comprising at least the following steps:
- installing a test support arm system according to one of claims 1 to 10 including at least one movably supported support arm at the desired site, and/or for usage for the same intended purpose as the support arm system which is to be designed;
- usage of the test support arm system;
- detecting respective load data of the test support arm system based on the use thereof, in particular by moving the support arm thereof;
- storing the detected load data of the test support arm system; and
- designing the support arm system (10) depending on the stored load data of the test support arm system.

## Revendications

1. Système de bras de support (10) pour un support mobile d'au moins un appareil médical, comprenant au moins un bras de support (18, 20, 22, 24) monté de manière mobile, qui est conçu pour le support du au moins un appareil médical, comprenant au moins un dispositif de détection de charge (26), qui est conçu pour détecter des données de charge respectives du système de bras de support (10) lors de son utilisation, dans lequel le système de bras de support (10) comprend plusieurs bras de support (18, 20, 22, 24) montés les uns à côté des autres, dans lequel le dispositif de détection de charge (26) est conçu pour détecter la charge séparément pour les bras de support respectifs (18, 20, 22, 24) et/ou leurs paliers respectifs, et au moins un dispositif de mémoire (46) qui est conçu pour stocker les données de charge détectées.

2. Système de bras de support (10) selon la revendication 1, dans lequel le au moins un dispositif de détection de charge (26) est conçu pour détecter les données de charge respectives du système de bras de support (10) en déplaçant le bras de support (18, 20, 22, 24).

3. Système de bras de support (10) selon la revendication 1 ou 2, dans lequel le système de bras de support (10) comprend en outre un dispositif d'évaluation (48) qui est conçu pour déterminer des nécessités de maintenance respectives pour le système de bras de support (10) en fonction des données de charge stockées et/ou pour déterminer les exigences structurelles pour la conception en fonction des données de charge stockées, en particulier sélectionnées dans le groupe constitué du type de palier, de la sélection du matériau des composants structurels, de l'épaisseur de paroi et de la structure de palier d'un système de bras de support supplémentaire pour la même utilisation.

4. Système de bras de support (10) selon l'une quelconque des revendications précédentes, dans lequel le dispositif de détection de charge (26) est conçu pour détecter les positions absolues respectives des bras de support respectifs (18, 20, 22, 24), pour détecter les positions relatives respectives des bras de support respectifs (18, 20, 22, 24) les uns par rapport aux autres, pour détecter les mouvements respectifs et/ou
pour détecter des accélérations des bras de support respectifs (18, 20, 22, 24), pour détecter des forces respectives, en particulier celles agissant sur les bras de support respectifs (18, 20, 22, 24), et/ou pour détecter les vibrations respectives, en particulier celles subies par les bras de support (18, 20, 22, 24) et/ou les paliers respectifs.

5. Système de bras de support (10) selon l'une quelconque des revendications précédentes, dans lequel le dispositif de détection de charge (26) présente au moins un capteur (30, 32, 34, 36).

6. Système de bras de support (10) selon la revendication précédente, dans lequel le dispositif de détection de charge (26) présente au moins un capteur (30, 32, 34, 36) associé à un bras de support (18, 20, 22, 24) et/ou à un palier.

7. Système de bras de support (10) selon l'une quelconque des revendications précédentes, dans lequel ledit au moins un capteur (30, 32, 34, 36) est conçu sous la forme d'un capteur de force, d'un capteur d'accélération, d'un capteur de torsion, d'un capteur de couple, d'un capteur de position, d'une jauge de contrainte, d'un capteur de butée, d'un capteur GPS, d'un capteur DGPS, d'un capteur de champ magnétique, d'un capteur de luminosité, d'un capteur à ultrasons, d'un gyroscope et/ou d'un capteur de pression.

8. Système de bras de support (10) selon l'une quelconque des revendications précédentes, dans lequel le dispositif de détection de charge (26), en particulier ses capteurs respectifs (30, 32, 34, 36), est ou sont reliés au dispositif de mémoire (46) par câble ou sans câble pour la transmission de données.

9. Système de bras de support (10) selon l'une quelconque des revendications précédentes, dans lequel le dispositif de stockage (46) est conçu comme serveur central.

10. Système de bras de support (10) selon la revendication 9, dans lequel le dispositif de stockage (46) est conçu pour stocker les données de charge détectées des dispositifs de détection de charge respectifs (26) de plusieurs systèmes de bras de support (10).

11. Système de détection de charge de bras de support avec plusieurs systèmes de bras de support (10) selon l'une quelconque des revendications précédentes et un dispositif de stockage (46) pour stocker de manière centralisée les données de charge de tous les systèmes de bras de support (10) et/ou un dispositif d'évaluation (48) pour évaluer de manière centralisée les données de charge de tous les systèmes de bras de support (10).

12. Procédé pour faire fonctionner un système de bras de support (10) selon l'une quelconque des revendications 1 à 10 pour un support mobile d'au moins un appareil médical, comprenant au moins les étapes suivantes consistant à :
- déplacer un bras de support mobile (18, 20, 22, 24) du système de bras de support (10) avec au moins un dispositif médical supporté sur celui-ci ;
- détecter les données de charge respectives du système de bras de support (10) lors de son utilisation, en particulier en déplaçant le bras de support (18, 20, 22, 24) ; et stocker des données de charge détectées.

13. Procédé selon la revendication 12, comprenant en outre au moins les étapes suivantes consistant à :
- déterminer les nécessités de maintenance respectives pour le système de bras de support (10) en fonction des données de charge stockées ; et/ou
- déterminer les exigences structurelles pour la conception d'un système de bras de support supplémentaires en fonction des données de charge stockées.

14. Procédé de conception d'un système de bras de support (10) pour un support mobile d'au moins un dispositif médical au moyen d'un bras de support (18, 20, 22, 24) monté mobile, comprenant au moins les étapes suivantes consistant à :
- installer un système de bras de support de test selon l'une quelconque des revendications 1 à 10 avec au moins un bras de support monté mobile sur le lieu d'utilisation souhaité et/ou pour une utilisation dans le même but que le système de bras de support à concevoir ;
- utiliser le système de bras de support de test ;
- détecter les données de charge respectives du système de bras de support de test lors de son utilisation, en particulier en déplaçant son bras de support ;
- stocker les données de charge détectées du système de bras de support de test ; et
- concevoir le système de bras de support (10) en fonction des données de charge stockées du système de bras de support de test.
